# EUROPEAN PATENT APPLICATION

(11) **EP 1 374 856 A1**
(43) Date of publication of application: **02.01.2004**
(21) Application number: 03013185.8
(22) Date of filing: 12.06.2003
(51) Int. Cl.: A61K 9/10, A61K 31/56, A61K 47/44, A61P 11/02

(54) **Oily thixotropic nasal spray**

(30) Priority: 18.06.2002 EP 02013289
(71) Applicant: Impetus AG, 6302 Zug (CH)
(72) Inventor: Jaenicke, Christof, Dr., 13465 Berlin (DE)
(74) Representative: Maucher W., Dipl.-Ing.

(57) **Abstract**

The invention relates to an oily thixotropic preparation comprising an antihistamine, or a pharmaceutically acceptable salt thereof, and/or a glucocorticosteroid, or a pharmaceutically acceptable salt thereof, as such or for use in the therapeutic treatment of the human or animal body, in particular for use in the therapeutic treatment of nasal disorders, in particular allergic rhinitis, the use of such a preparation for the therapeutic treatment of nasal disorders; its use for the manufacture of a product for the therapeutic treatment of especially nasal disorders, and/or a method for the therapeutic treatment of especially nasal disorders comprising the administration of such a preparation to an animal or in particular to a human being. These formulations show a synergistic effect.

## Description

### Summary of the invention

The invention relates to an oily thixotropic antihistamine and/or glucocorticosteroid preparation as such or for use in the therapeutic treatment of the human or animal body, in particular for use in the therapeutic treatment of nasal disorders, in particular allergic rhinitis, the use of such a preparation for the therapeutic treatment of nasal disorders, its use for the manufacture of a product for the therapeutic treatment of especially nasal disorders, and/or a method for the therapeutic treatment of especially nasal disorders, comprising the administration of such a preparation to an animal or in particular to a human being.

### Background to the invention

Numerous organic compounds, in particular with larger molar mass, and many inorganic compounds, in suitable dispersion media and, if required, with the use of specific methods, form colloidal solutions, known as sols. Under certain conditions their viscosity may become so great that a gelatinous mass is formed (coagulation), although the volume of the dispersion agent is predominant by far in comparison with the dispersion phase. These gelatinous masses, deriving from sols, are referred to as gels. It is possible, for example, by warming up, to reverse the formation of the gel and revert to the sol state (peptisation).

One particular type of conversion of sols into gels and vice-versa is thixotropy. Certain sols become gelatinous rapidly, if they are allowed to stand quietly. Under the effect of mechanical forces (shear forces), for example due to shaking, pressing through a nozzle, or stirring, the viscosity of these gels will be reduced to such an extent, however, that they must again be regarded as sols. If the system subjected to shear forces is again allowed to stand in peace, the high initial viscosity will within a short time be restored, and the specimen will again become a gel. The sol-gel conversion and vice-versa can be repeated as often as may be wished. This can be demonstrated very illustratively with a sol of magnesium trisilicate. Thixotropic processing is based on the fact that the dispersal phase, when standing, acquires a certain scaffold structure due to the forces of attraction between the particles, which is, however, so labile that it will at least in part be destroyed again by mechanical vibrations. In the resting phase, the scaffold structures are restored, and the viscosity rises again.

Thixotropic gels have already been applied in a series of formulations, in which situation, however, only their use in the cosmetics sector or, in the pharmaceutical sector, their function as a depot for pharmaceutical active substances. Examples of nasal formulations can be found in the International Application WO 98/00178, where the thixotropic gels are described as suspending media for solid particles of certain specific medicamentously active substances, due to their depot effect for these substances on the nasal mucous membrane, and in the International Application WO 94/05330, where likewise thixotropic basic formulations are used for the formulation of certain specific pharmaceutical active substances, with the aim of reducing their flowback and outflow from the nasal area, and so provide for a longer dwell time of the active substances in the nose. EP 0 780 127 describes combinations of a glucocorticosteroid and the antihistamines cetirizine, loratidine and/or azelastine, but not compositions with antihistamines alone. None of these applications specifically mentions oily components.

The causes of allergic rhinitis, a disorder of the nasal mucous membrane, are in principle known. Mainly, an immune reaction takes place, which, due to specific mediators, incurs local and systemic effects in the patient. Primarily, tissue mast cells are attacked, which feature, *inter alia,* non-specific and specific receptors in the outer cell membrane (for immunoglobulin E, for example). The bonding of allergens to these receptors leads to the release of histamine and other inflammatory mediators, such as leucotrienes and prostaglandins. As a consequence of the effect of these mediators, the musculature of the vessels becomes slack, the permeability of the vessels and the membrane increases, and constriction of the bronchial musculature occurs. The symptoms of allergic rhinitis follow from these reactions: Local rubor and swelling of the nasal mucosa and of the conjunctiva, as well as increased emergence of fluid into the tissue and resultant increased secretion in the nose and eyes. Systemic effects may also occur, such as hypotonic states in the cardiovascular system.

This cascade of effects is triggered primarily by exogenic agents, such as pollen, house dust, animal hair, foodstuff constituents, pharmaceutical preparations or mites, or by antibodies which were themselves formed in the organism as a reaction to pollen or other foreign substances. This is a disorder which is very frequently encountered: A survey conducted in 1992/1993 of 2,400 school students indicated that 13.5 % had suffered from hayfever in the previous year. Pollen allergies are the leaders in atopic disorders.

The therapy for allergic rhinitides, in particular for allergic rhinoconjunctivitis (pollinosis, "hayfever"), with aqueous nasal secretion, blocked respiration due to obstruction, itching nose, sneezing attacks combined with inflammation and itching irritation of the eyes, as well as tear flow, in addition, frequently, a general feeling of malaise, weakness, fatigue, exhaustion, in times of high allergy burden up to the level of complaints in other organ systems, bronchial hyperreactivity with asthma symptoms, exacerbation (worsening) of atopical dermatitis, or also gastrointestinal disorders) is based on three main principles: Allergen restriction, medicamentous therapy (symptomatic only), and specific immunotherapy (hyposensitisation).

Allergen restriction, which takes causal effect, is intended to avoid or reduce the exposure of the patient to allergens, in order to avoid contact with mast cells. This includes measures in the everyday conduct of life, such as keeping windows and doors closed, fitting pollen filters in air-conditioning systems, no sports practised in the open air, holidays in other climatic zones, etc. This however requires a high level of compliance on the part of the patient and his surroundings. The method is effective prophylactically , especially. In medication therapy, modern oral and topical pharmaceutical products are used, such as disodium chromoglycate (DNCG) and Nedocromil for topical administration, systemic or topical antihistamines, systemic or topical glucocorticoids, and α-sympathicomimetics and anticholinergics. Even in the event of slight symptoms, combinations of different medications are, as a rule, used nowadays. As further therapy, hyposensitisation is effected by regular injection of preparations which contain allergen extracts, and is based on the education of the immune system to a moderate reaction.

Medicamentous therapy is considerably elaborate and expensive, and as a general rule the attempt must also be made to reduce allergen exposure. Oversensitivity reactions are possible. The causally effective hyposensitisation requires in particular a high level of diagnostic effort, is elaborate, and (because of the regular injections over an extended period) is a burden on the patient and does not demonstrate any sustained or reliably reproducible effect.

Against this background, the objective of the present invention is to provide new preparations which make additional forms of therapy (including prophylaxis) possible, which show, as far as possible, low side-effects, for example by necessitating lower doses of active ingredients, and which are effective in a simple manner (in particular prophylactically), and which make possible in particular a complete or extensive allergen restriction.

### General description of the invention

It has now been found, surprisingly, that the administration of an oily thixotropic gel comprising an antihistamine and/or a glucocorticosteroid, or a pharmaceutically acceptable salt of an antihistamine and/or a glucocorticosteroid, provides a positive effect against nasal disorders, and against allergic rhinitis in particular. The effect takes place very fast and for a prolonged time and locally.

On the one hand, without wishing to be bound solely to this explanation, it appears that the fast effect against the allergens is supported by a "mechanical" barrier function (in particular due to the oily components contained and/or the viscosity of the gel), the allergens being no longer able to proliferate directly on the nasal mucous membrane due to the largely uniform distribution of the gel on the mucous membrane, and they therefore do not come in contact with the mast cells. As a result, the allergic reactions described hereinbefore can be eliminated entirely or diminished. The protective function can take place directly after administration. Thus, the thixotropic preparation, due to its prompt blocking effect, can help to bridge the time between administration and the onset of the therapeutic activity of the antihistamine and/or the glucocorticosteroid, thus providing a useful extra effect

On the other hand, an additional synergistic effect is surprisingly observed if the thixotropic formulation contains an antihistaminic and/or a glucocorticosteroid agent. The use of thixotropic gels on an aqueous basis for the nasal administration of some specific active agents is known (see above). However, due to the prompt protection of the nasal mucosa against the allergens by the thixotropic preparation according to the invention formulated on an oily basis, the clinical efficacy of the antihistamine and/or glucocorticosteroid drug is remarkably improved. The effective dose can be reduced and the duration of the therapeutic action of antihistaminic and/or glucocorticosteroid drugs can be prolongated due to the adhesiveve properties of the oily thixotropic formulation. The solubility in the oily phase adds to the pharmacologically and pharmacokinetically advantageous efffects.

Resulting from the synergistic actions of these mechanisms - especially protection of the nasal mucosa against the allergens by an oily film and improved local action of the antihistaminics at lower doses - the period during which the patients remain free of symptoms can, for example, be doubled. This means that the regular frequency of administration of antihistaminics and/or glucocorticosteroids can be expected to be remarkably reduced, allowing for prolonged administration intervals, e.g. once daily.

As a further result of reduction of the individual dose, a better pharmacologic profile regarding side effects, e.g. systemic effects, like sedation, may be observed.

### Detailed description of the invention

The invention relates to an oily thixotropic preparation comprising an antihistamine and/or a glucocorticosteroid, or a pharmaceutically acceptable salt of an antihistamine and/or a glucocorticosteroid, in particular oily thixotropic nose drops comprising an antihistamine and/or a glucocorticosteroid, or a pharmaceutically acceptable salt an antihistamine and/or a glucocorticosteroid or, especially, an oily thixotropic nasal spray comprising an antihistamine and/or a glucocorticosteroid, or a pharmaceutically acceptable salt of an antihistamine and/or a glucocorticosteroid, for use in the therapeutic treatment of the human or animal body, for preference of warm-blooded animals or especially of human beings, and in particular for use in the therapeutic treatment of nasal disorders, especially of allergic rhinitis, the use of such a preparation for the therapeutic treatment of nasal disorders in particular, its use for the manufacture of a pharmaceutical or medical product for the therapeutic treatment of nasal disorders in particular, and/or a method for the therapeutic treatment of nasal disorders in particular, which includes the administration of such a preparation to an animal, preferably to a warm-blooded animal, or most especially to a human being.

The terms generally used hereinbefore and hereinafter have for preference the meanings indicated below, unless indicated otherwise, whereby more specific meanings may be used independently of one another in preferred embodiments of the present inventions instead of the general definitions, these more specific significances describing especially preferred embodiments of the invention.

Where the term "at least one" or "one or more" occurs hereinbefore and hereinafter, this signifies in particular one to ten, for preference one to three, and in particular one or, further, two of the features enumerated, such as components. Where ranges are indicated, such as weight percentage ranges, these include the limit values indicated; thus, for example, "between X and Y" signifies "from and including X up to and including Y".

Fractions of components are indicated as "% by weight" (percentage by weight), related to the finished (complete) preparation.

An antihistamine is, in particular, selected from the group consisting of Azelastine, Levocabastine, Cetirizine, Loratidine, Terfenadine and Fexofenadine, including ; leucotriene antagonists, such as Prankulast, Zafirlukast, or Montelukast; or, provided that salt-forming groups are present, pharmaceutically acceptable salts of the aforesaid substances and compounds; or mixtures thereof. Included in the term "antihistamine" are these salts, the pure compounds and/or racemates or essentially pure enantiomers where asymmetry is possible. Azelastine, or a pharmaceutically acceptable salt thereof, is especially preferred. A formulations comprising only an antihistamine and no glucocorticosteroid, or pharmaceutically acceptable salts thereof, is specifically preferred.

A glucocorticosteroid is, in particular, selected from the group consisting of Budesonide, Flunisolide, Fluocortinbutyl, Fluticasone, Fluticason-17-propionate, Mometasone, and Mometasone furoate, or, provided that salt forming groups are present, pharmaceutically acceptable salts thereof, or mixtures of the compounds or salts.

An antihistamine is present, related to the finished preparation, for preference overall in an amount of 0.005 to 10 % by weight, for preference from 0.05 to 1 % by weight. A glucocorticosteroid is present, related to the finished preparation, for preference in an amount of 0.001 to 2 % by weight, for preference from 0.01 to 1 % by weight.

In a broader aspect of the invention, also other therapeutic agents may be present in addition to the antihistamine(s), glucocorticosteroids, such as Flutikasone.

The term "product" is to be understood to mean in particular a pharmaceutical product in the sense of a formulation, for preference a pharmaceutical product, whereby this term is not restricted to pharmaceutical products suitable for registration, or a medical product or fictitious pharmaceuticals ("Geltungsarzneimittel", that is, products which the German Drug Law subsumes under medical products, though they do not fall under the strict term "Arzneimittel" (drugs)).

The term "thixotropic preparation" is to be understood to mean such that feature, when subjected to shear forces (shaking, pressing through a nozzle, stirring, or the like), a low viscosity (sol state), suitable in particular for use as nose drops or in particular as a nasal spray, for preference in the range from 1 to 40, in particular from 5 to 20 Pa*s (= kg/(m × s)), while by contrast when in a state of rest feature a high viscosity, for preference in the range from 40 to 100 Pa*s. The measurement is effected by means of a rotation viscosimeter.

The oily thixotropic preparation according to the invention comprises for preference at least four components: (A) An aqueous basis, (B) at least one oily component (which is a very important component), (C) at least one gel-forming agent for thixotropic gels (components having the effect of creating suspensions, which may lend the preparation thixotropic properties) and (D) at least one antihistamine and/or at least one glucocorticosteroid, or a pharmaceutically acceptable salt of an antihistamine and/or a glucocorticosteroid. With particular preference, just these four components are present.

The basis components (A) to (C) referred to hereinbefore and hereinafter are in particular selected from among those such as are listed in pharmacopoeia, e.g. in the US Pharmacopoeia National Formulary, the Pharmacopoea Europea, the Pharmacopoea Helvetica, the British Pharmacopoeia, or the German Pharmacopoeia, or supplements, such as by way of decrees.

As an aqueous basis, water is particularly suitable, to which (already in the aqueous basis as such, in mixture with other constituents of a preparation according to the invention, or as a constituent of the finished preparation) other water-soluble components can be added, such as diols or polyols, in particular ethylene glycol, 1,2 propylene glycol, a sugar alcohol such as sorbite, hexite, or mannite, or in particular glycerine, as additives (e.g. supporting and stabilizing the formation of the gel). Water is present for preference in a proportion of 10 to 80, for preference from 30 to 70, e.g. 40 to 60 % by weight, and stabilizing additives may be contained in this component in a quantity in the range from, for preference, 0.1 to 10 % by weight, related to the finished composition, respectively. The water proportion is suitable in particular for regulating the basic viscosity of the thixotropic gel.

A large number of products are suitable as oily components, in particular:
(i) Hydrocarbons, e.g. mineral oils, in particular paraffin, paraffin oil (in particular white paraffin oil, low-viscosity or in a broader embodiment high-viscosity paraffin oil), purcellin oil or perhydrosqualene, or further hard paraffin or vaseline, whereby low-viscosity paraffin oil is preferred; or
(ii) preferably vegetable oils, e.g. almond oil, groundnut oil, wheatgerm oil, rape oil, linseed oil, apricot oil, walnut oil, palm oil, pistachio oil, sesame oil, poppyseed oil, pine oil, castor oil, soya oil, avocado oil, cocoa oil, hazelnut oil, olive oil, grapeseed oil, rice oil, maize germ oil, peach-kernel oil, coffee oil, Jojoba oil, sunflower oil, thistle oil, cocoa butter or the like, or the hydrated, polyoxyethylated, polyoxy- or hydrated polyoxyderivatives or fractionated derivatives thereof, whereby castor oil is especially preferred; or mixtures of two or more of these components.

Further examples of additional or alternative oily components which are more preferred than hydrocarbons but less preferred than vegetable oils are animal oils, saturated or non-saturated esters, higher alcohols and/or silicone oils, or mixtures of two or more of these components.

To reinforce the retention on the mucous membranes, waxes, together with one or more oils such as those defined hereinbefore, may form part of an oily component of this type, such as carnauba wax, Cera alba, Cera flava, Cera chinesis, Cera japonica, Candelilla wax, microcrystalline wax, wool wax or okozerite.

Particularly preferred are the oily components referred to under (ii), especially castor oil.

The oily component has for preference a proportion by weight in the finished preparation from 10 to 80, in particular from 40 to 70 % by weight.

As gel formers for thixotropic gels consideration may be given to, for example:
(a) Organic suspension media, such as
   (i) polysaccharides, in particular cellulose (in particular microcrystalline cellulose, such as Avicel® (FMC Corporation, Philadelphia, USA) or cellulose derivatives, such as carboxymethyl cellulose or the salts thereof (in particular alkaline metal salts, such as sodium salts), alone or in mixtures with microcrystalline cellulose, methyl cellulose, guar gum, traganth or dextrine esters, such as is disclosed in EP 0 736 545, which is hereby incorporated by reference in this respect:
   (ii) polymer compounds which do not belong to the polysaccharides, in particular polyvinyl alcohols, polyacrylates, such as polyacrylic acid or polymethacrylate (cross-bonded in particular), polyacrylate block copolymers with alternating hydrophilic and hydrophobic blocks, such as, in particular, Hypane hydrogels (Kingston Technology Inc., N.Y., USA), such as in EP 0 836 847, which is hereby incorporated in this respect; or
   (iii)special organic gel formers, such as oxyethylenated (= polyoxyethylenated) polyol fatty acid esters, in particular alkyl- (especially methyl-) monosaccharide-C₆-C₂₄-fatty acid esters, or mixtures thereof (alone or in mixtures with appropriate non-oxyethylenated polyol-fatty acid esters, in particular alkyl- (especially methyl-) monosaccharide-C₆-C₂₄-fatty acid esters, or mixtures thereof, preferably methylglucoside-sesquistearate (= mixture of the mono and distearate), oxyethylenated with ethylene oxide (in particular 2 to 50, and especially 20 mol), with 20 mol ethylene oxide added, available for example under the brandname "Glucamate SSE 20®" from Amerchol Corp. (Du Pont), Edison, NJ, USA; CAS No. 68389-70-8) or a mixture thereof with methylglucoside-sesquistearate (available for example under the brandname "Glucamate SSE 20® " (Amerchol); CAS No. 68389-70-8)), for preference in the proportion by weight of 10:1 to 1:10, in particular from 3:1 to 1:3, e.g. of 1:1; others are the mixtures described in WO 94/14822 of metal salts of phosphoric acid diesters and organopolysiloxanes, which are derivatised with a polyvalent metal salt of a phosphoric acid diester containing silicon, which is why this patent application is hereby incorporated in this respect; or
(b) Inorganic suspension media, such as kaolin clays forming colloids, in particular (especially hydrated) metal oxide silicates, such as bentonite, or (for preference highly dispersed colloidal) silicon dioxide in dried or, for preference, hydrated form, three-layer clay materials such as smectite, in particular synthetic smectites with trioctahedric co-ordinated cations, manufactured from magnesium silicates and alkali cations, or suitable colloidal magnesium-aluminium silicates; or synthetic hectorite clays such as Laponite® Laporte, London, Great Britain);
or mixtures of two or more of these components.

Particularly preferred are the "special organic gel formers" referred to under (iii),.

The gel formers are present for preference in a proportion by weight, related to the finished preparation, from 0.1 to 15 % by weight, for preference (in particular in the case of organic gel formers) from 0.2 to 10 % by weight, such as, especially, from 0.25 to 4 % by weight.

The preparation can also include other additives, such as, especially, binders, surface-active substances ("surfactants"), conservation agents, colouring agents, flavouring substances, pH regulators, regulators of osmotic activity, or the like.

Binders (in addition to the gel formers) may be, for example, dextrines, hydroxypropyl cellulose, hydroxypropyl methyl cellulose, polyvinyl pyrrolidon, gelatines, pectin, starch, or also sodium carboxymethyl cellulose, which in part also have gel-forming properties. These may be present in proportions of, for preference, up to 10 % by weight, for preference from 0.1 to 3 % by weight, related to the finished preparation.

Surfactants may be present in the preparation in quantities of up to 2 % by weight, for example from 0.2 to 0.8 % by weight. For preference, anionic detergents, such as sulphonates, are used, e.g. alkylbenzol sulphonates, alkanesulphonates, Alfa-olefin sulphonates, 2-acyloxy-alkan-1-sulphonates, or fatty acid monoglyceride sulphates; sulphates, such as alkyl sulphates, polyoxyethyl sulphates, fatty acid monoglyceride sulphates, or the salts thereof, for example with alkali metals, ammonia or ammonium compounds; cationic detergents, e.g. quaternary ammonium compounds, such as alkylarylether dimethylbenzalkonium halogenides, alkyltrimethyl ammonium chlorides, alkyldimethyl ethylammonium halides, alkylpyridinium halogenides, alkyldimethyl ethylammonium halides, alkylpyridinium halogenides, alkylimidazolium halogenides, alkyldimethyl dichlorobenzyl ammonium halogenides, acylcolamonoformylinether pyridinium halogenide or alkylarylmethyl pyridinium halogenides; non-ionic detergents, such as polyethylene oxide condensates of alkylphenolene, ethylene oxide condensation products, derived from the reaction of ethylene oxide with the product from propylene oxide and ethylene diamine, the condensation products of aliphatic alcohols with ethylene oxide, or amide detergents which contain an ammonium, monoethanolamino, diethanolamino, or other alkanolamide group; zwitterionic detergents, such as aliphatic quaternary compounds containing ammonium and phosphonium or sulphonium groups; or amphoteric detergents, such as aliphatic secondary or tertiary amines. Preferred are the surface-active compounds referred to in WO 94/05330, as a result of which this application is incorporated here in this respect by reference.

Preservation agents are in particular complex formers, such as ethylenediamine tetraaceticacid, antioxidants such as ascorbic acid, butylated hydroxyanisol (BHA) or butylated hydroxytoluene (BHT) or other preservation agents such as methyl, ethyl, propyl, or butyl esters of 4-hydroxy-benzoic acid (Parabene), benzoic acid, cetrimide, cetyltrimethyl ammonium chloride, sorbinic acid or thiomersal, or the like. Preservation agents may be present, for example, in weight proportions (related to the finished preparation) of up to 1 % by weight, and in particular in the range from 0.005 to 0.5 % by weight. Preferably, preservation agents are omitted in order to avoid negative effects on the self-purifying mechanisms of the nasal mucosa.

As colouring agents, use may be made, for example, of toxicologically accetable substances such as beta-cerotin, Erythrosin, Gelborange S, Indigotin, or Tartrazin. By the addition of colouring agents of this type, can be examined, for example, to determine whether the preparations according to the invention are distributed over the entire nasal mucous membrane. Colouring agents may be present, for example, in the weight proportions (related to the finished preparation) of up to 1 % by weight, and in particular from 0.001 to 0.2 % by weight.

Flavouring substances are for preference not among the flavouring substances which belong to the etheric oils or plant extracts, but such as fruit flavouring substances (e.g. fruit esters) or vanillin, which may be present, for example, as up to 1 % by weight of the finished preparation, and in particular between 0.001 and 0.5 % by weight.

As pH regulators, use may be made of buffer additives, e.g. phosphate buffers or buffers on a citric acid basis. For preference these serve to adjust the pH value to between 3 and 9, and in particular between 5 and 8, and may be present in concentrations of up to, in particular, less than 120 mM, for example 20 to 100 mM.

As regulators of osmotic activity, use may be made, for example, of sugars or sugar alcohols (which can be used at the same time as additives) or, in particular, sodium chloride, for example (in particular for sodium chloride) in a concentration of up to 0.9 % (% by weight).

Alkyl (in case of sulphonates, alkan) has for preference 1 to 30, for example 1 to 18, carbon atoms, and can be unbranched or single-branched or multiple-branched, provided that sufficient carbon atoms are present. Aryl has for preference 6 to 14, and in particular 6 to 10, ring carbon atoms, and has a single ring or a double or multiple ring, and is unsubstituted or single-substituted or multiple-substituted, in particular by alkyl, such as methyl or ethyl, carboxy, esterified carboxy, halogen, such as fluorine, chlorine, bromine or iodine, or also other substitutents; phenyl or naphthyl is preferred, unsubstituted or substituted as above. Acyl is for preference alkylcarbonyl or hydroxyalkylcarbonyl, in each case defined with alkyl as above, arylcarbonyl, for preference with aryl as defined above, or arylalkylcarbonyl, defined with aryl and alkyl as above.

If salt-forming groups (anionic, such as carboxy, sulphonyl, or sulphate) or cationic groups (such as amino) are present in the components or active substances referred to above, they may be also be present (in whole or in part) as (especially pharmaceutically acceptable) salts, in the case of anionic groups, for example, as alkali metal salts, alkaline-earth metal salts, ammonium salts, zinc salts, or tin salts, and in the case of cationic groups, for example as salts of organic or inorganic acids, such as of hydrogen halides hydrocarbons, sulphuric acid, phosphoric acid, organic sulphones or sulphates or carboxylic acids, such as acetic acid, or, if both anionic and cationic groups are present, as inner salts.

The person skilled in the art is aware, or can determine in a simple experimental manner, which of the components referred to can be combined with one another to form a preparation according to the invention, without mutually negative influences arising (precipitation, absence of thixotropic properties, or the like).

The term "therapeutic treatment" of the human or animal body, of the human in particular, also includes prophylaxis (in particular in the case of the preparations according to the invention "for use in treatment") and the alleviation of symptoms, although not cosmetic treatments. For preference, this relates to therapeutic treatment, and in particular prophylaxis or alleviation, of disorders of the upper nasal passages, in particular of the nasopharynx (hereinbefore and hereinafter "nasal disorders), and in particular for the therapy and prophylaxis of allergic processes located therein, such as, in particular, allergic rhinitis, and especially pollinosis (for preference, since with low night-time exposure to pollen, treatment during the day is sufficient and also only requires seasonal therapy), as well as house dust or animal hair allergy.

The oily thixotropic gels according to the invention are used in particular as nasal drops (usually in bottles with pipettes) or, for preference, as nasal sprays. For nasal sprays, portable spray devices are used in particular.

Examples of suitable (for preference activated by finger pressure) portable spray devices (nasal dispensers), which comprise a storage container, a spray pump, and for preference means for nebulization (if desired, also loose stirring elements, such as small metal pellets or rods, which contribute to the shaking up effect), are known, for example from German Patent DE 19 542 959, which describes both distribution pumps as well as simple squeeze bottles in combination with a storage container. Pre-compression pumps, such as are described in WO 98/00178, can also be used, such as the VP7/100S pump (Perfect Valois VP7), from Valois SA, France. Very well suited are also the "3-K Pumpe" (3-K Pump) or the COMOD system from AeroPump GmbH, Hofheim/Taunus, Germany. These types of pumps, especially in view of silver components, allow to avoid preservation agents. For preference, the dosage unit amounts to 1 to 50 ml, in the case of portable storage containers for nasal sprays for preference 3 to 30 ml, in particular 5 to 20 ml. The invention relates in particular to an oily thixotropic preparation comprising an antihistamine, or a pharmaceutically acceptable salt thereof, inside a portable spray device of this type.

Administration can be effected in any desired manner. In the case of nasal administration, the quantity suitable for treatment of the disorders referred to, administered per spray burst, in particular the quantity of the preparation released with a nasal spray per spraying action, is for preference between 3 and 200 µl, and in particular between 50 and 150 µl, e.g. approximately 140 µl.

Administration takes place once or usually several times daily, in particular at intervals of a few hours, for example at intervals of 2 to 8, and in particular of 3 to 6 hours; for preference starting directly after waking, and, if protection is also required at night, for the last time immediately before going to sleep. In especially preferred embodiments, only one to three daily treatments are used, especially one or one in the morning and one in the evening.

The preparations according to the invention are manufactured according to conventional and known methods, which include in particular the mixing of the components, if required step by step and/or under movement and/or heating of the fluid, for example by stirring. In order to arrive at the pharmaceutical preparations according to the invention, a start is made for preference with sterile initial components and work is carried out under sterile conditions; incomplete interim stages, which do not yet contain all the components, are sterilised and/or the preparations according to the invention are subjected at the end to a sterilisation procedure by suitable usual methods. The determination of the thixotropy is effected as described above, and a check can therefore easily be carried out as to whether the mixtures manufactured with the above components fall among the thixotropic gels to be used according to the invention. The enumeration of the components (A) to (D) upwards or downwards is not to be understood such that these components must be added one after another in sequence, and in particular not that water and the additives of component A are mixed separately; rather, the components can be mixed in any conceivable sequence, in particular the customary sequence and manner, for the manufacture of preparations according to the invention.

Use for the manufacture of a product for the therapeutic treatment of nasal disorders comprises in particular the appropriate procurement of the product (e.g. as a pharmaceutical product in the broader or narrower sense); in other words, the manufacture of the preparation, its introduction in particular into the spray device, its packing, and the corresponding provision of instructions for use in therapeutic treatment, for example by way of a package insert and/or printings on the package.

Preferred embodiments of the invention are described hereinafter, whereby, whenever mention is made of a "preparation", this means an oily thixotropic preparation comprising an antihistamine, or a pharmaceutically acceptable salt thereof, as such or especially for use in therapeutic (including prophylactic) treatment of the human or animal body, in particular of the human, especially against the specific disorders referred to hereinbefore and hereinafter; and, whenever the use of a preparation of this nature is referred to, this means use in the therapeutic treatment referred to, for the manufacture of a product for therapeutic treatment as referred to, or a method or process for therapeutic treatment of this nature, with the use of the preparation, unless defined otherwise.

The invention relates in particular to a preparation or the use thereof for the therapeutic treatment of disorders of the nose and pharynx, in particular of the nasopharynx, especially of allergic rhinitis and of pollinosis.

The preparation comprises for preference (A) an aqueous basis, in particular in a proportion of 10 to 80 % by weight, for preference from 30 to 70, more preferably from 40 to 60 % by weight, related to the finished preparation, whereby in the aqueous phase, for preference as further water-soluble component, one or more additives are present, in particular one or more di- or polyols, especially glycerine, in such a quantity that the preparation contains a total of 0.1 to 10 % by weight thereof; (B) an oily component, in particular one or more vegetable oils, whereby, in addition, waxes may also belong to the oily component; in particular castor oil; whereby the oily component amounts to 10 to 80 % by weight, for preference 40 to 70, of the preparation; (C) one or more gel formers for thixotropic gels, selected in particular from (a) organic suspension media, such as (i) polysaccharides, (ii) polymer compounds not belonging to the polysaccharides, or, especially, (iii) special organic gel formers, such as oxyethylenated polyol fatty acid esters, preferably alkyl-, especially methyl-monosaccharide-C₆-C₂₄ fatty acid esters or mixtures thereof, alone or in a mixture with corresponding non-oxyethylenated polyol fatty acid esters, preferably alkyl-, especially methyl monosaccharide-C₆-C₂₄ fatty acid esters, or mixtures thereof, in particular methylglucoside sesquistearate (= mixture of the monostearate and distearate), oxyethylenated with preferably 2 to 50, in particular 20 mol ethylene oxide, or a mixture thereof with methylglucoside sesquistearate, whereby the gel formers comprise a total of 0.1 to 15 % by weight, for preference 0.2 to 10 % by weight, in particular 0.25 to 4 %, of the complete preparation, and (b) inorganic suspension media (which may be present in the same amounts); and (D) one or more antihistamines and/or one or more glucocorticosteroids, or pharmaceutically acceptable salts of said antihistamine(s) and/or glucocorticosteroid(s)n particular azelastine, loratidine and/or cetirizine, or a pharmaceutically acceptable salt, respectively; in addition, further additives, such as, in particular, binders (for preference up to 10 % by weight, in particular 0.1 to 3 % by weight), surfactants (for preference up to 1 % by weight, in particular 0.2 to 0.8 % by weight), preservation agents (for preference up to 1 % by weight, in particular from 0.005 to 0.5 % by weight), colouring agents (for preference up to 1 % by weight, in particular from 0.001 to 0.2 % by weight), flavouring substances (for preference up to 1 % by weight, in particular from 0.001 to 0.5 % by weight), pH regulators (in particular 120 nM or less), or regulators of osmotic activity (for preference up to 0.9 % by weight), may be present in a less preferred embodiment of the invention, whereby, if salt-forming groups are present in the components, these may also be present (in whole or in part) as salts; and whereby, in a particularly preferred preparation, only the components listed under (A), (B), (C) and (D) are present (and/or pharmaceutically acceptable salts thereof if not already mentioned). Preparations of the type referred to above, which comprise the special organic gel formers referred to under (B) (iii), in particular those referred to therein as preferred, are a special embodiment of the invention.

The preparation relates in particular to oily thixotropic nasal drop or especially to an oily thixotropic nasal spray, each commprising an antihistamine or a pharmaceutically acceptable salt thereoff, for preference in a portable spray device, for preference a nasal dispenser, which comprises a storage container, a spray pump, and a device for nebulization.

The use (for preference for therapeutic (in particular prophylactic) treatment) of a preparation according to the invention is effected in particular in the form of a nasal spray, which is used for preference several times a day, in particular beginning shortly after waking and last used immediately before going to sleep, at intervals of several hours, in particular from 2 to 8, for preference from 3 to 6 hours, and for preference in a volume of between 3 and 500 µl, in particular between 50 and 150 µl, per nostril.

The invention also relates to a method (= process) for the therapeutic (including prophylactic) treatment of disorders, which comprises administering a preparation according to the invention to an animal or in particular to a human being requiring such treatment (or consists in particular of the administration thereof), for preference for prophylaxis or alleviation, especially against disorders of the upper respiratory passages, preferably of the nasopharynx, in particular for the therapy and prophylaxis of allergic processes taking place in that area, such as, in particular, allergic rhinitis, and especially of pollinosis in particular, but also of house dust or animal hair allergy, in particular in the form of nasal drops or for preference as a nasal spray, in particular in the quantities referred to above as preferential.

The invention also relates to oily thixotropic nasal drops or in particular an oily thixotropic nasal spray, each comprising an antihistamine or a pharmaceutically acceptable salt thereof and/or a glucocorticosteroid or a pharmaceutically acceptable salt thereof, preferably an antihistamine or a pharmaceutically acceptable salt thererof; preferably in each case as defined as being preferred above, particularly in the preferred amounts mentioned above, in particular in a portable spray device, especially as described above, as such, especially for the therapeutic treatment, in particular for the prophylaxis or alleviation, of disorders of the upper respiratory passages, especially of the nasopharynx, especially for the therapy and prophylaxis of allergic processes in that area, such as allergic rhinitis in particular, and especially pollinosis, as well as house dust or animal hair allergy, of an animal, in particular of a human being, for preference comprising a quantity of said nasal drops or said nasal spray effective against said disorders.

Especially preferred embodiments of the invention are also provided in the claims or in particular in the examples, where in particular the preparations listed as such are new and are an embodiment of the invention.

Examples: The following examples serve as illustrations of the invention, without restricting the scope thereof.

### Example 1: Oily thixotropic nose gel containing loratidine

A thixotropic gel is manufactured with the mixing of the components according to standard methods; see Rudolf Voigt, "Pharmazeutische Technologie" (Pharmaceutical Technology), Deutscher Apothekerverlag (publishing house), 2000. In brief, the stirring process is pursued in a homogenizer for a short period of time at high speed (3000 to 6000 revolutions per minute, which leads to the formation of gel with cross-linking), with the result that the following composition of the resultant gel is obtained:

| Constituent class | Constituent | Proportion % by weight |
|---|---|---|
| Oily constituent | Castor oil | 44.0 % |
| Gel formers | Glucate SS | 1.5 % |
| | Glucamate SSE | 1.5 % |
| Additive | Glycerine | 2.5 % |
| Antihistamine | Loratidine | 0.3 % |
| Basis | Water | 49.2 % |

Glucate SS is methyl glucoside sesquistearate. Glucamate SSE 20 is a methyl glucoside sesquestearate, which is polyoxyethylenated with 20 mol ethylene oxide. Both are available from Amerchol Corp., Edison, NJ, USA (now Union Carbide).

The castor oil is a product according to the Europaean Pharmacopoea. The preparation is suitable for use as nasal drops, but is also used in particular as a nasal spray.

### Example 2: Oily thixotropic nasal gel containing azelastine

A thixotropic gel is manufactured with the mixing of the components according to the standard method as referred to under Example 1, so that the following composition of the resultant gel is obtained:

| Constituent class | Constituent | Proportion % by weight |
|---|---|---|
| Oily constituent | Castor oil | 44.0 % |
| Gel formers | Glucate SS | 1.5 % |
| | Glucamate SSE | 1.5 % |
| Additive | Glycerine | 2.5 % |
| Antihistamine | Azelastine • HCl | 0.1% |
| Basis | Water | 49.4 % |

### Example 3: Oily thixotropic nasal gel containing fluticasone

A thixotropic gel is manufactured with the mixing of the components according to the standard method as referred to under Example 1, so that the following composition of the resultant gel is obtained:

| Constituent class | Constituent | Proportion % by weight |
|---|---|---|
| Oily constituent | Castor oil | 44.0 % |
| Gel formers | Glucate SS | 1.5 % |
| | Glucamate SSE | 1.5 % |
| Additive | Glycerine | 2.5 % |
| Glucocorticosteroid | fluticasone | 0.05 % |
| Basis | Water | 49.45 % |

### Example 4: Nasal spray

One of the gels described in any one of Examples 1 to 3 is filled into a conventional spray device (optionally, also equipped with a metal pellet which is freely movable under shaking, to promote peptisation in the storage chamber). The spraying device is a combination of storage container with 20 ml content and a dosage pump with a nose adapter for nebulization. A suitable spray device of this type, which also has the advantage that no anti-microbial active substances are required in the compositions used for filling, since silver is provided in the outlet nozzle for the disinfection of the spray residue which may flow back, is one of the models with the designation "3-K Pumpe" (3-K pump) or COMOD system (AeroPump GmbH, nebulization pumps, Hofheim/Taunus, Germany); in this case, 140 µl is administered per spray action. After brief shaking, the gel present in the storage container is liquified (sol state), and can be applied as a spray mist with no trouble, using the administration pump located on the storage container. Once the mist impinges on the surface of the mucosa, the settlement phase beings, and the thixotropic gel solidifies again and forms a film on the surface of the nasal mucous membrane. This forms a layer which is permeable for allergens only with difficulty or is impermeable for them, with the result that they do not come in contact with mast cells.

### Example 5: Use with patients

In the scope of a field study, the efficacy of a nasal spray spraying device according to Example 4, containing a thixotropic gel according to one of Examples 1 to 3, is tested on patients with pollinosis. The study is conducted on 25 patients. In the first instance they are asked about the seasonal occurrence of symptoms daily for a period of one week, for example as to whether they suffer from swelling of the nose, secretion from the nasal mucous membrane, sneezing, or the like. The same patients then use a nasal spray according to Example 4, with the gel according to the invention from Example 1. The administration is effected for the first time after getting up in the morning, and thereafter at intervals of a few hours (e.g. 3-4), until going to sleep. After shaking the gel to cause it to change to the sol state, a spray volume of 100 µl is administered to each nostril with the head inclined slightly backwards. During the week of treatment, the patients are asked daily about their symptoms, for example as to whether they are suffering from swelling of the nose, increased secretion from the nasal mucous membrane, sneezing, conjunctivitis or the like. Most of the patients present symptoms which are clearly reduced in comparison with the week free of therapy, and also present a subjective improvement in their symptoms.

Comparison is possible by using gels without oily component.

### Example 6: Reduction of hayfever by oily thixotropic basis without antihistamine

According to the methods for determining test scores published in the FDA Guidance for Industry - Allergic Rhinitis: Clinical Development Programs for Drug Products, the diminuation of the allergic reactions described hereinbefore associated with hayfever can be demonstrated using an especially equipped clima chamber (Fraunhofer Institut für Allergologie und Klimatologie, Germany). Patients are exposed to grass pollen (4000 pollen/cbm air). A nasal spray according to Example 3, however without the antihistamine, is used. The exposition time is four hours and the usual symptoms of hayfever are significantly reduced as demonstrated in the subsequent table:

| Test Item | Control | | Treated | | Significance | |
|---|---|---|---|---|---|---|
| | Means | S.D. | Means | S.D. | p | |
| Running nose | 25.5 | 6.0 | 21.3 | 7.9 | 0.0042 | <5% |
| Stuffed up nose | 24.2 | 9.8 | 19.8 | 9.2 | 0.0269 | <5% |
| Itching nose | 17.5 | 7.3 | 13.7 | 7.7 | 0.0540 | |
| Sneezing | 15.0 | 7.5 | 7.6 | 4.8 | 0.0002 | < 0.1 % |

Looking on the time course of symptoms, it is observed that after 3 to 4 hours the intensity of the symptoms increased, probably due to the transport of the protecting film by the mucociliary system.

However, in the presence of an antihistamine the latter will prolong the effect of this protective film due to ist own mechanism of action, resulting in a synergistic efffect.

## Claims

1. An oily thixotropic preparation comprising an antihistamine or a pharmaceutically acceptable salt therof and/or a glucocorticosteroid or a pharmaceutically acceptable salt thereof.

2. A preparation according to claim 1, comprising
(A) an aqueous basis, in particular in a proportion of 10 to 80 % by weight, for preference from 30 to 70, more preferably from 40 to 60 % by weight, related to the finished preparation, whereby the aqueous phase, for preference as additional water-soluble components, contains one or more additives, in particular one or more di- or polyols, especially glycerine, in such a quantity that the preparation contains a total of 0.1 to 10 % by weight thereof;
(B) an oily component, in particular one or more vegetable oils or mixtures thereof, whereby, in addition, waxes may belong to the oily components; in particular castor oil; whereby the oily component constitutes 10 to 80 % by weight of the preparation, and for preference 40 to 70 % by weight;
one or more gel formers for thixotropic gels, selected in particular from (a) organic suspension media such as (i) polysaccharides, (ii) polymer compounds not belonging to the polysaccharides, or, in particular, (iii) special organic gel formers, for preference oxyethylenated polyol fatty acid esters, in particular alkyl-, especially methyl-, monosaccharide-C₆-C₂₄ fatty acid esters, or mixtures thereof (alone or in mixture with corresponding non-oxyethylenated polyol fatty acid esters, in particular alkyl-, especially methyl-, monosaccharide-C₆-C₂₄ fatty acid esters , or mixtures thereof), preferably methyl glucoside sesquistearate, oxyethylenated with 2 to 50, in particular 20 mol ethylene oxide, or a mixture thereof with methyl glucoside sesquistearate, whereby the gel formers constitute a total of 0.1 to 15 % by weight, for preference 0.2 to 10 % by weight, in particular 0.25 to 4 % of the complete preparation, and, further, (b) inorganic suspension media;
and an antihistamine, or a salt thereof, especially an antihistamine selected from the group consisting of cetirizine, loratidine or especially azelastine, or in each case a pharmaceutically acceptable salt thereof; and/or (less preferably) a glucocorticosteroid or a pharmaceutically acceptable salt thereof, especially fluticasone.
and in a broader embodiment, further additives, such as, in particular, binders (for preference up to 10 % by weight, and in particular 0.1 to 3 % by weight), surfactants (for preference up to 1 % by weight, in particular 0.2 to 0.8 % by weight), preservation agents (for preference up to 1 % by weight, in particular from 0.005 to 0.5 % by weight), colouring agents (for preference up to 1 % by weight, in particular from 0.001 to 0.2 % by weight), flavouring substances (for preference up to 1 % by weight, in particular between 0.001 and 0.5 % by weight), pH regulators (in particular 120 mM or less), or regulators of the osmotic activity (for preference up to 0.9 % by weight),
whereby, if salt-forming groups are present in the components, these may also be present (in whole or in part) as salts.

3. A preparation according to claim 2, wherein only the components listed under (A), (B), (C) and (D) (and/or salts thereof) are present.

4. A preparation as such, but in particular according to one of claims 1 to 3, comprising, preferably consisting solely of
(A) an aqueous basis, in particular in a proportion from 30 to 70 % by weight, especially from 40 to 60 % by weight, related to the finished composition, whereby, the aqueous phase, for preference as additional water-soluble components, contains one or more additives, in particular a di- or polyol, especially glycerine, in such a quantity that the preparation contains a total of 0.1 to 10 % by weight thereof;
(B) as oily component, one or more vegetable oils or mixtures thereof; whereby the oily component constitutes 10 to 80 % by weight of the preparation, for preference 40 to 70 % by weight;
gel formers for thixotropic gels, selected in particular from polyoxyethylated polyol fatty acid esters, for preference alkyl-, especially methyl-, monosaccharide-C₆-C₂₄ fatty acid esters, or mixtures thereof, alone or in a mixture with corresponding non-oxyethylenated polyol fatty acid esters, in particular alkyl-, especially methyl-, monosaccharide-C₆-C₂₄ fatty acid esters, or mixtures thereof; in particular methylglucoside sesquistearate, oxyethylated with 2 to 50 mol, especially 20 mol, ethylene oxide or a mixture thereof with methylglucoside sesquistearate, and further (b) inorganic suspension media; and
an antihistamine or a pharmaceutically acceptable salt thereof, especially cetirizine, loratidine or especially azelastine, or in each case a pharmaceutically acceptable salt thereof; and/or a glucocorticosteroid or a pharmaceutically acceptable salt thereof.

5. A preparation according to claim 4, or in particular according to one of claims 1 to 3, with the following composition: Castor oil 40 to 50 % by weight, for preference 44.0 % by weight; methylglucoside sesquistearate, oxyethylenated with 20 mol ethylene oxide 0.5 to 5 % by weight, for preference 1.5 % by weight; methylglucoside sesquistearate 0.5 to 5 % by weight, for preference 1.5 % by weight; glycerine 0.5 to 5 % by weight, for preference 2.5 % by weight; and water 40 to 60 % by weight, for preference 49.2 to 49.4 % by weight, and antihistamine, especially loratidine or azelastine, and/or (less preferably) a glucocorticosteroid; or in each case a pharmaceutically acceptable salt thereof, 0.05 to 1 % by weight, especially 0.1 to 0.3 % by weight.

6. A use of a preparation according to any one of claims 1 to 5 for the treatment of a nasal disorder.

7. The use according to claim 6, whereby the preparation is used as a nasal spray.

8. The use according to claim 6 or 7, whereby the disorder to be treated is allergic rhinitis, in particular pollinosis.

9. The use according to any one of claims 6 to 8, whereby the preparation is administered in a volume of 3 to 200 µl, in particular from 50 to 150 µl, per nostril, once or for preference several times a day, in particular at intervals of several hours, for example at intervals of 2 to 8, for preference of 3 to 6, hours, for preference beginning directly after waking and, if protection is also required at night, for the last time immediately before going to sleep.

10. The use of a thixotropic preparation free of active substances, or of all the components thereof, in accordance with any one of claims 1 to 5, for the manufacture of a pharmaceutical product for the treatment of nasal disorders.

11. The use according to claim 10, whereby the preparation is used for the manufacture of a nasal spray.

12. The use according to claim 10 or 11, whereby the disorder to be treated is allergic rhinitis, in particular pollinosis.

13. A method of treatment of a nasal disorder comprising administering to an individual in need of such treatment an amount of a preparation according to claim 1 or claim 5 that is effective in the treatment of said nasal disorder.
